# FASCICULE DE BREVET EUROPEEN

(11) **EP 1 299 371 B1**
(45) Date de publication et mention de la délivrance du brevet: **06.02.2008**
(21) Numéro de dépôt: 01967093.4
(22) Date de dépôt: 26.06.2001
(51) Int. Cl.: C07D 301/12

(54) **FABRICATION D'OXIRANNE AU MOYEN D'UN COMPOSE PEROXYDE**
HERSTELLUNG VON OXIRANEN MITTELS EINER PEROXI-VERBINDUNG
OXIRANE PRODUCTION USING A PEROXIDIZED COMPOUND

(30) Priorité: 28.06.2000 FR 0008355
(43) Date de publication de la demande: 09.04.2003
(73) Titulaire: SOLVAY (Société Anonyme), 1050 Bruxelles (BE)
(72) Inventeur: BALTHASART, Dominique, B-1120 Bruxelles (BE)
(74) Mandataire: Vande Gucht, Anne
(86) Numéro de dépôt international: PCT/EP2001/007273
(87) Numéro de publication internationale: WO 2002/000637

(56) Documents cités:
- EP-A- 0 659 473
- US-A- 5 849 937

## Description

La présente invention concerne un procédé de fabrication d'oxiranne par réaction entre une oléfine et un composé peroxydé en présence d'un catalyseur et d'un solvant. En particulier, elle concerne la fabrication d'oxyde de propylène (ou d'épichlorhydrine) par époxydation de propylène (ou de chlorure d'allyle) au moyen de peroxyde d'hydrogène en présence d'un catalyseur contenant du TS-1.

Il est connu de fabriquer de l'oxyde de propylène par réaction entre le propylène et le peroxyde d'hydrogène en présence de TS-1. Par exemple, dans la demande de brevet EP 0659473 il est décrit l'utilisation d'une tour catalytique. Alternativement, dans le brevet US 5,849,937 un tel procédé est réalisé dans plusieurs réacteurs disposés en série. Dans ce procédé connu, chaque réacteur de la série est alimenté en peroxyde d'hydrogène frais.

La demanderesse a constaté que, lorsque l'on alimente chaque réacteur en peroxyde d'hydrogène frais, il est impossible de convertir la totalité de la quantité mise en oeuvre de peroxyde d'hydrogène sans formation importante de sous-produits et donc d'avoir un rendement optimal.

La présente invention remédie à cet inconvénient en fournissant un nouveau procédé qui permet de convertir 100% de la quantité introduite de peroxyde d'hydrogène avec une faible formation de sous-produits et sans pour autant diminuer la vitesse de réaction.

A cet effet, l'invention concerne un procédé de fabrication d'oxiranne par réaction d'une oléfine avec un composé peroxydé en présence d'un catalyseur et d'un solvant dans au moins deux réacteurs disposés en série dont chacun contient une partie du catalyseur, selon lequel on introduit dans un premier réacteur une première partie de l'oléfine, le solvant et l'entièreté du composé peroxydé, on y effectue une époxydatlon de la première partie de l'oléfine pour former une première partie de l'oxiranne, on soutire de ce réacteur un milieu comprenant la première partie d'oxiranne formé, le solvant, le composé peroxydé non consommé et éventuellement l'oléfine non convertie, on introduit dans un réacteur ultérieur le milieu et une autre partie de l'oléfine, on y effectue une époxydation de l'autre partie de l'oléfine au moyen du composé peroxydé non consommé provenant du premier réacteur pour former une autre partie de l'oxiranne, et on recueille l'autre partie de l'oxiranne ainsi formé.

Une des caractéristiques essentielles de la présente invention réside dans le fait que le composé peroxydé soit uniquement introduit dans le premier réacteur. Le ou les réacteurs ultérieurs ne sont donc pas alimentés en composé peroxydé frais mais seulement avec le composé peroxydé qui est présent dans le milieu provenant du réacteur précédent et qui n'a pas été consommé dans ce réacteur précédent. En général, on introduit également de l'eau avec le composé peroxydé dans le premier réacteur. Le fait de ne pas ajouter du composé peroxydé dans le(s) réacteur(s) ultérieur(s) permet de consommer 100 % de la quantité totale de composé peroxydé mise en oeuvre sans pour autant diminuer la vitesse de réaction par rapport à un procédé utilisant la même quantité totale de composé peroxydé mais dans lequel chaque réacteur est alimenté en composé peroxydé frais.

Dans le procédé selon l'invention on met en oeuvre une installation comprenant au moins deux réacteurs d'époxydation disposés en série et reliés entre eux, à l'exception d'au moins deux zones de réaction successives disposées dans un seul réacteur. Il s'agit donc de réacteurs distincts. Chaque réacteur est alimenté en oléfine. Le composé peroxydé et le solvant sont uniquement introduits dans le premier réacteur. Chaque réacteur contient une partie du catalyseur qui ne quitte pas ce réacteur. Lorsque le catalyseur est présent sous la forme d'un lit fixe, il n'est en général pas nécessaire de prendre des précautions pour maintenir le catalyseur dans le réacteur. En variante, le catalyseur peut être présent sous la forme de particules dont une partie au moins est à l'état fluidisé par un courant liquide ou par agitation mécanique ou par un gaz. Lorsqu'on utilise un courant liquide, il est recommandé de prévoir une zone de dégagement surmontant le lit fluide pour arrêter les particules de catalyseur qui sont en mouvement et/ou de prévoir un filtre à la sortie du réacteur.

L'installation peut évidemment comprendre plus de deux réacteurs connectés en série. Dans ce cas, le premier réacteur de la série est alimenté avec l'oléfine, le composé peroxydé et le solvant et chaque réacteur ultérieur est alimenté avec l'oléfine et le milieu provenant du réacteur précédent de la série. On utilise de préférence 3 réacteurs en série.

Dans le procédé selon l'invention on consomme généralement, dans le premier réacteur, au moins 50 % de la quantité totale de composé peroxydé mise en oeuvre dans le premier réacteur. Les meilleurs rendements sont obtenus lorsqu'au moins 70 % sont consommés dans le premier réacteur. Le plus souvent, on consomme au plus 99 % dans le premier réacteur, et de préférence au plus 85 %. Le restant est consommé dans le(s) réacteur(s) ultérieur(s).

Dans le procédé selon l'invention, on utilise de préférence des réacteurs de dimension identique. Ceci permet de permuter la fonction des réacteurs lorsque le catalyseur désactivé d'un réacteur est remplacé par du catalyseur frais ou régénéré sans perturber le fonctionnement de l'installation (fonctionnement dit "en carrousel").

Une première forme de réalisation particulièrement avantageuse du procédé selon l'invention consiste à utiliser le catalyseur sous forme de particules dont une partie au moins se trouve à l'état fluidisé, comme décrit dans la demande de brevet de la demanderesse déposée le même jour que la présente demande de brevet et intitulée "Procédé de fabrication d'oxiranne en présence d'un catalyseur sous forme de particules" (dont le contenu est incorporé par référence). Dans ce cas, il est recommandé de prévoir un filtre au travers duquel passe le milieu sortant du premier réacteur avant d'être introduit dans le réacteur ultérieur. Cette forme de réalisation permet d'obtenir une dispersion homogène du catalyseur dans le milieu réactionnel d'époxydation, un bon échange thermique et donc un contrôle aisé de la température de réaction.

Dans une deuxième forme de réalisation du procédé selon l'invention, on soumet le milieu entrant dans le réacteur ultérieur d'abord à une détente avant d'être introduit dans le réacteur ultérieur. Cette forme convient particulièrement bien lorsque l'époxydation est réalisée sous pression ou en présence d'un composé gazeux. Ce composé gazeux peut être l'oléfine-même (par exemple le propylène) ou un gaz inerte que l'on introduit dans le milieu réactionnel d'époxydation pour permettre d'entraîner l'oxiranne et de le sortir du réacteur, comme décrit dans la demande de brevet WO 99/48883 de la demanderesse.

Dans une troisième forme de réalisation du procédé selon l'invention, on soumet le milieu entrant dans le réacteur ultérieur d'abord à un traitement de séparation de l'oxiranne formé avant d'être introduit dans le réacteur ultérieur. Cette forme de réalisation a pour but de séparer l'oxiranne du milieu réactionnel d'époxydation le plus vite possible dès sa formation afin d'éviter la formation de sous-produits par hydrolyse ou par alcoolyse (méthanolyse lorsque le méthanol est utilisé comme solvant) de l'oxiranne formé. Cette forme de réalisation présente donc l'avantage de conduire à une sélectivité élevée. Le traitement de séparation est de préférence une distillation, comme décrit dans la demande de brevet de la demanderesse déposée le même jour que la présente demande de brevet et intitulée "Procédé de fabrication d'oxiranne comprenant la séparation de l'oxiranne du milieu réactionnel" (dont le contenu est incorporé par référence).

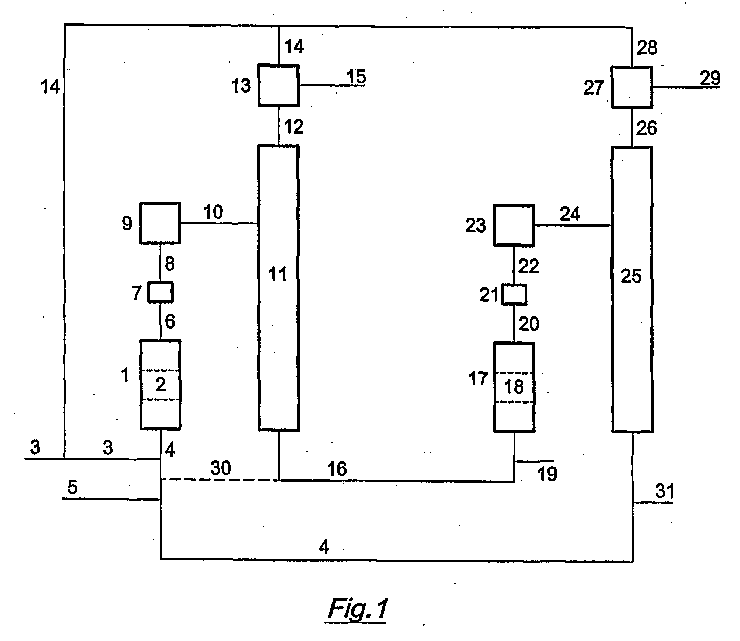

Une forme de réalisation préférée du procédé selon l'invention est schématisée dans la figure 1. Dans cette forme préférée, le premier réacteur 1 contient une partie du catalyseur, de préférence en lit fluide 2. Le réacteur 1 est alimenté en une première partie de l'oléfine par le conduit 3 et ensuite par le conduit 4, en composé peroxydé par le conduit 5 en ensuite par le conduit 4, et en solvant par le conduit 4 provenant d'une autre partie de l'installation qui est décrite plus loin. Dans le premier réacteur, la première partie de l'oléfine réagit avec le composé peroxydé en présence du catalyseur pour former une première partie de l'oxiranne. Le milieu sortant du réacteur 1 via le conduit 6 contient le solvant, la première partie de l'oxiranne, le composé peroxydé non consommé et l'oléfine non convertie. Ce milieu passe à travers un filtre 7, et est envoyé via le conduit 8 dans le récipient 9 où il est soumis à une détente. Le milieu est ensuite transporté via le conduit 10 dans une colonne à distiller 11. En tête de cette colonne à distiller 11, on récupère un mélange d'oxiranne et d'oléfine non convertie. Ce mélange est envoyé via le conduit 12 dans un condenseur 13 qui sépare l'oxiranne de l'oléfine non convertie. L'oléfine non convertie est recyclée dans le réacteur 1 via les conduits 14, 3 et 4. La première partie d'oxiranne est recueillie comme produit fini via le conduit 15. En pied de la colonne à distiller 11, on recueille un milieu contenant le solvant, le composé peroxydé non consommé dans le réacteur 1 et éventuellement une partie de l'oléfine non convertie. Ce milieu, dont une partie peut éventuellement être recyclée dans le réacteur 1 via le conduit 30, est transporté via le conduit 16 dans un deuxième réacteur 17 contenant une autre partie du catalyseur; de préférence à l'état d'un lit fluide 18. Le deuxième réacteur 17 est alimenté en une deuxième partie de l'oléfine via le conduit 19. Dans le deuxième réacteur 17, la deuxième partie de l'oléfine réagit avec le composé peroxydé non consommé provenant du premier réacteur en présence du catalyseur 18 pour former une deuxième partie de l'oxiranne. Les conditions dans le deuxième réacteur 17 sont de préférence telles que la totalité du composé peroxydé provenant du premier réacteur soit consommée. Le milieu sortant du réacteur 17 via le conduit 20 contient alors le solvant, la deuxième partie de l'oxiranne et l'oléfine non convertie. Ce milieu passe à travers un filtre 21, et est envoyé via le conduit 22 dans le récipient 23 où il est soumis à une détente. Le milieu est ensuite transporté via le conduit 24 dans une deuxième colonne à distiller 25. En tête de cette colonne à distiller 25, on récupère un mélange de la deuxième partie d'oxiranne et d'oléfine non convertie. Ce mélange est envoyé via le conduit 26 dans un condenseur 27 qui sépare l'oxiranne de l'oléfine non convertie. L'oléfine non convertie est recyclée dans le réacteur 1 via les conduits 28, 14, 3 et 4. La deuxième partie d'oxiranne est recueillie comme produit fini via le conduit 29. En pied de la colonne à distiller 25, on recueille le solvant qui est recyclé via le conduit 4 dans le premier réacteur 1, et un effluent aqueux qui est évacué via le conduit 31.

Le catalyseur utilisé dans le procédé selon l'invention contient généralement une zéolite comme élément actif, et de manière préférée, une zéolite au titane. Par zéolite au titane, on entend désigner un solide contenant de la silice qui présente une structure cristalline microporeuse de type zéolite et dans laquelle plusieurs atomes de silicium sont remplacés par des atomes de titane. La zéolite au titane présente avantageusement une structure cristalline de type ZSM-5, ZSM-11, ZSM-12, MCM-41, ZSM-48. Elle peut aussi présenter une structure cristalline de type zéolite bèta, de préférence exempte d'aluminium. Les zéolites présentant une bande d'absorption infrarouge à environ 950-960 cm⁻¹ conviennent bien. Les zéolites au titane de type silicalite sont préférées. Celles répondant à la formule xTiO₂(1-x)SiO₂ dans laquelle x est de 0,0001 à 0,5 de préférence de 0,001 à 0,05, sont performantes. Des matériaux de ce type, connus sous le nom de TS-1, présentent une structure zéolitique cristalline microporeuse analogue à celle de la zéolite ZSM-5.

Le catalyseur utilisé dans le procédé selon l'invention se présente avantageusement sous la forme de particules obtenues par extrusion comme décrit dans la demande de brevet WO 99/28029 de la demanderesse, ou par un procédé en spray comme décrit dans la demande de brevet WO 99/24164 de la demanderesse. Le contenu de ces deux demandes de brevet est incorporé par référence dans celle-ci.

Le solvant mis en oeuvre dans le procédé selon l'invention peut être choisi parmi les alcools aliphatiques saturés, linéaires ou branchés. Le solvant alcoolique contient généralement jusqu'à 10 atomes de carbone, de préférence de 1 à 6 atomes de carbone. On peut citer à titre d'exemples le méthanol et l'éthanol. Le méthanol est préféré.

La quantité de solvant mise en oeuvre dans le premier réacteur est généralement d'au moins 25 % en poids du milieu réactionnel liquide présent dans le premier réacteur, en particulier d'au moins 40 % en poids, par exemple d'au moins 50 % en poids. Cette quantité ne dépasse habituellement pas 99 % en poids, en particulier pas 95 % en poids.

Le rapport molaire entre les quantités d'oléfine et de composé peroxydé engagées dans le procédé selon l'invention est généralement d'au moins 0.1, en particulier d'au moins 0,2, et de préférence d'au moins 0,5. Ce rapport molaire est le plus souvent d'au plus 100, en particulier d'au plus 50 et de préférence d'au plus 25.

Le procédé selon l'invention peut être continu ou discontinu.

Dans le procédé selon l'invention, lorsqu'il est réalisé en continu, le composé peroxydé est généralement mis en oeuvre dans le premier réacteur en une quantité d'au moins 0,005 mole par heure et par gramme de catalyseur présent dans le premier réacteur, en particulier, d'au moins 0,01 mole. La quantité de composé peroxydé est habituellement inférieure ou égale à 25 moles et, en particulier, inférieure ou égale à 10 moles. Une préférence est montrée pour une quantité de composé peroxydé supérieure ou égale à 0,03 mole et inférieure ou égale à 2,5 mole.

Dans le procédé selon l'invention le composé peroxydé est avantageusement mis en oeuvre sous forme d'une solution aqueuse. En général, la solution aqueuse contient au moins 2 % en poids de composé peroxydé, en particulier au moins 5 % en poids. Elle contient le plus souvent au maximum 90 % en poids de composé peroxydé, en particulier 70 % en poids.

La température de la réaction entre l'oléfine et le composé peroxydé peut varier de 10 à 125 °C. Dans une variante avantageuse telle que décrite dans la demande de brevet EP99/08703 de la demanderesse, elle est supérieure à 35 °C pour remédier à la désactivation progressive du catalyseur. La température peut être supérieure ou égale à 40 °C et de préférence supérieure ou égale à 45 °C. Une température supérieure ou égale à 50 °C est tout particulièrement préférée. La température de réaction est de préférence inférieure à 100 °C.

Dans le procédé selon l'invention, la réaction entre l'oléfine et le composé peroxydé peut avoir lieu à pression atmosphérique. Elle peut également se dérouler sous pression. Généralement, cette pression n'excède pas 40 bar. Une pression de 20 bar convient bien en pratique.

Les composés peroxydés qui peuvent être utilisés dans le procédé selon l'invention sont les composés peroxydés contenant une ou plusieurs fonctions peroxyde (-OOH) qui peuvent libérer de l'oxygène actif et capables d'effectuer une époxydation. Les composés peroxydés inorganiques donnent de bons résultats. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Lorsqu'on utilise du peroxyde d'hydrogène, il peut être intéressant de mettre en oeuvre dans le procédé selon l'invention une solution aqueuse de peroxyde d'hydrogène à l'état brut, c'est-à-dire non épurée. Par exemple, on peut mettre en oeuvre une solution obtenue par simple extraction avec de l'eau substantiellement pure du mélange issu de l'oxydation d'au moins une alkylanthrahydroquinone (procédé appelé "procédé AO auto-oxydation") sans traitement ultérieur de lavage et/ou de purification. Ces solutions brutes de peroxyde d'hydrogène contiennent généralement de 0,001 à 10 g/l d'impuretés organiques exprimées en COT (Carbone Organique Total). Elles contiennent habituellement des cations métalliques (tels que des métaux alcalins ou alcalino-terreux, comme le sodium) et des anions (tels que les phosphates, nitrates) en des teneurs de 0,01 à 10 g/l.

Dans une autre variante du procédé, on peut mettre en oeuvre une solution de peroxyde d'hydrogène produite par synthèse directe à partir d'oxygène et d'hydrogène en présence de méthanol.

L'oxiranne qui peut être préparé par le procédé selon l'invention est un composé organique comprenant un groupement répondant à la formule générale :

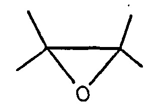

L' oxiranne contient généralement de 2 à 10 atomes de carbone, de préférence de 3 à 6 atomes de carbone. Les oxirannes qui peuvent être préparés de manière avantageuse par le procédé selon l'invention sont le 1,2-époxypropane et le 1,2-époxy-3-chloropropane. L'oxiranne préféré est le 1,2-époxypropane.

Les oléfines qui conviennent bien dans le procédé selon l'invention contiennent généralement de 2 à 10 atomes de carbone et de manière préférée, 3 à 6 atomes de carbone. Le propylène, le butylène et le chlorure d'allyle conviennent bien. Le propylène et le chlorure d'allyle sont préférés. Le propylène est tout particulièrement préféré.

Dans le procédé selon l'invention il peut s'avérer intéressant de contrôler le pH de la phase liquide. Par exemple, il peut être intéressant de maintenir le pH de la phase liquide lors de la réaction entre l'oléfine et le composé peroxydé à une valeur de 4,8 à 6,5, par exemple par addition d'une base (hydroxyde de sodium) au milieu d'époxydation, comme recommandé dans la demande de brevet WO 99/48882 de la demanderesse (dont le contenu est incorporé par référence dans la présente demande de brevet). Cette base peut être introduite dans un seul réacteur (par exemple, le premier réacteur) ou dans plusieurs réacteurs. Elle est de préférence introduite dans chaque réacteur.

La réaction entre l'oléfine et le composé peroxydé peut s'effectuer en présence d'un sel tel que le chlorure de sodium, comme décrit dans la demande de brevet WO EP99/08703 de la demanderesse (dont le contenu est incorporé par référence dans la présente demande de brevet). Ce sel peut être introduit dans un seul réacteur (par exemple, le premier réacteur) ou dans plusieurs réacteurs. Il est de préférence introduit dans chaque réacteur.

Il peut être avantageux d'introduire l'oléfine à l'état dilué dans un ou plusieurs alcanes. Par exemple, on peut introduire dans les réacteurs d'époxydation un fluide contenant l'oléfine et également au moins 10 % (en particulier 20%, par exemple au moins 30 %) en volume d'un ou plusieurs alcanes. Par exemple, dans le cas du propylène, celui-ci peut être mélangé avec au moins 10 % en volume de propane lorsqu'on introduit dans le réacteur le propylène non converti recyclé. Il peut également s'agir d'une source de propylène incomplètement épurée en propane.

Les exemples qui suivent sont destinés à illustrer la présente invention sans toutefois en limiter la portée.

Les exemples 1 et 2 ont été calculés au moyen du logiciel ASPEN PLUS® de la société ASPEN TECHNOLOGY INC. à l'aide des paramètres cinétiques de la réaction déterminés sur base des essais expérimentaux décrits et des équilibres liquide-vapeur disponibles dans la littérature.

### Exemple comparatif 1

Dans cet exemple, la synthèse de l'oxyde de propylène est effectuée dans 2 réacteurs en série avec séparation intermédiaire de l'oxyde de propylène formé au premier réacteur dans une colonne de rectification. L'H2O2 est alimenté pour moitié au premier réacteur et pour moitié au second réacteur.

326.5 kmol/h de peroxyde d'hydrogène accompagné de 1100 kmol/h d'eau sont divisées en deux fractions égales contenant chacune 163.25 kmol/h de peroxyde d'hydrogène et 550 kmol/h d'eau ; la première fraction est mélangée à 1500 kmol/h de méthanol, à 250 kmol/h de propylène et à la fraction recyclée du pied de la colonne de rectification sous une pression suffisante pour solubiliser tout le propylène à la température de réaction Le mélange réactionnel est introduit en continu à 70°C dans un réacteur méthodique contenant 600 kg de catalyseur. Le réacteur est maintenu à 70 °C par un système de réfrigération adéquat.

L'effluent du réacteur est dirigé vers une colonne de rectification contenant 50 plateaux théoriques (y compris condenseur et bouilleur) ; l'alimentation est effectuée au niveau du 10^{ème} plateau théorique (compté à partir du condenseur) ; la colonne est opérée à 1.1 bar absolu (pression de tête de colonne) ; la température de tête de colonne est maintenue à 40°C (distillat partiellement vaporisé) ; le taux de reflux molaire est fixé à 1 ; le débit total de distillat est fixé à 600 kmol/h.

Le mélange soutiré en pied de colonne, appauvrit en oxyde de propylène, est divisé en deux fractions, la première qui contient 90% volume du mélange est recyclée à l'alimentation premier réacteur ; la seconde qui contient le solde du mélange obtenu en pied de la colonne de rectification est mélangée à 200 kmol/h de propylène à une pression suffisante pour solubiliser l'entièreté du propylène à la température de réaction et introduit en continu à 70°C dans un second réacteur méthodique contenant 820 kg de catalyseur et maintenu à 70°C par un système de réfrigération adéquat.

L'effluent du second réacteur contient 24.5 kmol/h de peroxyde d'hydrogène non converti, 209.4 kmol/h d'oxyde de propylène et 87.7 kmol/h de sous-produits (methoxypropanol et propanediol principalement) ; le distillat de la colonne contient 56.2 kmol/h d'oxyde de propylène ; le rendement en C3 atteint 64.1 % pour un taux de conversion du peroxyde d'hydrogène de 92.4 %.

### Exemple 2 (conforme à l'invention)

Dans cet exemples, la synthèse de l'oxyde de propylène est effectuée dans 2 réacteurs en série avec séparation intermédiaire de l'oxyde de propylène formé au premier réacteur.

326.5 kmol/h de peroxyde d'hydrogène accompagné de 1100 kmol/h d'eau sont mélangé à 1500 kmol/h de méthanol à 250 kmol/h de propylène et à la fraction recyclée du pied de la colonne de rectification sous une pression suffisante pour solubiliser tout le propylène à la température de réaction Le mélange réactionnel est introduit en continu à 70°C dans un réacteur méthodique contenant 600 kg de catalyseur. Le réacteur est maintenu à 70 °C par un système de réfrigération adéquat.

L'effluent du réacteur est dirigé vers une colonne de rectification contenant 50 plateaux théoriques (y compris condenseur et bouilleur) ; l'alimentation est effectuée au niveau du 10ème plateau théorique (compté à partir du condenseur) ; la colonne est opérée à 1.1 bar absolu (pression de tête de colonne) ; la température de tête de colonne est maintenue à 40°C (distillat partiellement vaporisé) ; le taux de reflux molaire est fixé à 1 ; le débit total de distillat est fixé à 600 kmol/h.

Le mélange soutiré en pied de colonne, appauvrit en oxyde de propylène, est divisé en deux fractions, la première qui contient 90% volume du mélange est recyclée à l'alimentation premier réacteur ; la seconde qui contient le solde du mélange obtenu en pied de la colonne de rectification est mélangée à 200 kmol/h de propylène à une pression suffisante pour solubiliser l'entièreté du propylène à la température de réaction et introduit en continu à 70°C dans un second réacteur méthodique contenant 820 kg de catalyseur et maintenu à 70°C par un système de réfrigération adéquat.

L'effluent du second réacteur contient 1.9 kmol/h de peroxyde d'hydrogène non converti, 256.5 kmol/h d'oxyde de propylène et 62.2 kmol/h de sous-produits (methoxypropanol et propanediol principalement) ; le distillat de la colonne contient 88.4 kmol/h d'oxyde de propylène ; le rendement en C3 atteint 78.6% pour un taux de conversion du peroxyde d'hydrogène de 99.4%.

## Revendications

1. - Procédé de fabrication d'oxiranne par réaction d'une oléfine avec un composé peroxydé en présence d'un catalyseur et d'un solvant dans au moins deux réacteurs disposés en série dont chacun contient une partie du catalyseur, selon lequel on introduit dans un premier réacteur une première partie de l'oléfine, le solvant et l'entièreté du composé peroxydé, on y effectue une époxydation de la première partie de l'oléfine pour former une première partie de l'oxiranne, on soutire de ce réacteur un milieu comprenant la première partie d'oxiranne formé, le solvant, le composé peroxydé non consommé et éventuellement l'oléfine non convertie, on introduit dans un réacteur ultérieur lé milieu et une autre partie de l'oléfine, on y effectue une époxydation de l'autre partie de l'oléfine au moyen du composé peroxydé non consommé provenant du premier réacteur pour former une autre partie de l'oxiranne, et on recueille l'autre partie de l'oxiranne ainsi formé.

2. - Procédé selon la revendication 1, dans lequel 50 à 99 % de la quantité totale de composé peroxydé mise en oeuvre dans le premier réacteur sont consommés dans le premier réacteur, le restant étant consommé dans le(s) réacteur(s) ultérieur(s).

3. - Procédé selon la revendication 1 ou 2, dans lequel tous les réacteurs sont de dimension identique.

4. - Procédé selon l'une quelconque des revendications 1 à 3, dans lequel le catalyseur est présent dans chaque réacteur sous forme de particules dont une partie au moins se trouve à l'état fluidisé.

5. - Procédé selon la revendication 4, dans lequel le milieu sortant du premier réacteur passe à travers un filtre avant d'être introduit dans le réacteur ultérieur.

6. - Procédé selon l'une quelconque des revendications 1 à 5, dans lequel le milieu entrant dans le réacteur ultérieur est d'abord soumis à une détente avant d'être introduit dans le réacteur ultérieur.

7. - Procédé selon l'une quelconque des revendications 1 à 6, dans lequel le milieu entrant dans le réacteur ultérieur est d'abord soumis à un traitement de séparation de l'oxiranne formé avant d'être introduit dans le réacteur ultérieur.

8. - Procédé selon la revendication 7, dans lequel le traitement de séparation est une distillation.

9. - Procédé selon l'une quelconque des revendications 1 à 8, dans lequel l'oxiranne est l'épichlorhydrine, l'oléfine est le chlorure d'allyle, le composé peroxydé est le peroxyde d'hydrogène, le solvant est le méthanol, et le catalyseur contient du TS-1.

10. - Procédé selon l'une quelconque des revendications 1 à 8, dans lequel l'oxiranne est l'oxyde de propylène, l'oléfine est le propylène, le composé peroxydé est le peroxyde d'hydrogène, le solvant est le méthanol et le catalyseur contient de TS-1.

11. - Procédé selon l'une quelconque des revendications précédentes, dans lequel aucune quantité substantielle de solvant n'est ajoutée entre le premier et le second réacteur.

12. - Procédé selon l'une quelconque des revendications précédentes, dans lequel aucune quantité substantielle supplémentaire de solvant n'est ajoutée dans le second réacteur.

13. - Procédé selon l'une quelconque des revendications précédentes, dans lequel trois réacteurs en série sont utilisés.

## Claims

1. Process for manufacturing oxirane by reaction of an olefin with a peroxide compound in the presence of a catalyst and a solvent in at least two reactors arranged in series, each of which contains a portion of catalyst, according to which a first portion of the olefin, the solvent and all of the peroxide compound are introduced into a first reactor, an epoxidation of the first portion of the olefin is carried out therein in order to form a first portion of the oxirane, a medium comprising the first portion of the oxirane formed, the solvent, the unconsumed peroxide compound and possibly the unconverted olefin is removed from this reactor, the medium and another portion of the olefin are introduced into a subsequent reactor, an epoxidation of the other portion of the olefin is carried out therein using the unconsumed peroxide compound obtained from the first reactor, in order to form another portion of the oxirane, and the other portion of the oxirane thus formed is collected.

2. Process according to Claim 1, in which 50% to 99% of the total amount of peroxide compound used in the first reactor is consumed in the first reactor, the remainder being consumed in the subsequent reactor(s).

3. Process according to Claim 1 or 2, in which all the reactors are of identical size.

4. Process according to any one of Claims 1 to 3, in which the catalyst is present in each reactor in the form of particles, at least some of which are in fluidized form.

5. Process according to Claim 4, in which the medium leaving the first reactor passes through a filter before being introduced into the subsequent reactor.

6. Process according to any one of Claims 1 to 5, in which the medium entering the subsequent reactor is first subjected to a depressurization before being introduced into the subsequent reactor.

7. Process according to any one of Claims 1 to 6, in which the medium entering the subsequent reactor is first subjected to a treatment to separate out the oxirane formed before being introduced into the subsequent reactor.

8. Process according to Claim 7, in which the separation treatment is a distillation.

9. Process according to any one of Claims 1 to 8, in which the oxirane is epichlorohydrin, the olefin is allyl chloride, the peroxide compound is hydrogen peroxide, the solvent is methanol and the catalyst contains TS-1.

10. Process according to any one of Claims 1 to 8, in which the oxirane is propylene oxide, the olefin is propylene, the peroxide compound is hydrogen peroxide, the solvent is methanol and the catalyst contains TS-1.

11. Process according to any one of the preceding claims, in which no substantial amount of solvent is added between the first and second reactor.

12. Process according to any one of the preceding claims, in which no substantial additional amount of solvent is added in the second reactor.

13. Process according to any one of the preceding claims, in which three reactors in series are used

## Patentansprüche

1. Verfahren zur Herstellung von Oxiran durch Umsetzung eines Olefins mit einer Peroxidverbindung in Gegenwart eines Katalysators und eines Lösungsmittels in mindestens zwei hintereinandergeschalteten Reaktoren, die jeweils einen Teil des Katalysators enthalten, bei dem man einen ersten Teil des Olefins, das Lösungsmittel und die gesamte Peroxidverbindung in einen ersten Reaktor einträgt, darin eine Epoxidation des ersten Teils des Olefins durchführt, wobei ein erster Teil des Oxirans gebildet wird, aus diesem Reaktor ein Medium, das den ersten Teil des gebildeten Oxirans, das Lösungsmittel, die nicht verbrauchte Peroxidverbindung und gegebenenfalls nicht umgesetztes Olefin enthält, abzieht, das Medium und einen anderen Teil des Olefins in einen nachfolgenden Reaktor einträgt, darin eine Epoxidation des anderen Teils des Olefins mit der nicht verbrauchten Peroxidverbindung aus dem ersten Reaktor durchführt, wobei ein anderer Teil des Oxirans gebildet wird, und den anderen Teil des so gebildeten Oxirans gewinnt.

2. Verfahren nach Anspruch 1, bei dem 50 bis 99% der Gesamtmenge der im ersten Reaktor verwendeten Peroxidverbindung im ersten Reaktor verbraucht werden und der Rest in dem nachfolgenden Reaktor bzw. den nachfolgenden Reaktoren verbraucht wird.

3. Verfahren nach Anspruch 1 oder 2, bei dem die Reaktoren die gleiche Größe haben.

4. Verfahren nach einem der Ansprüche 1 bis 3, bei dem der Katalysator in jedem Reaktor in Form von zumindest teilweise fluidisierten Teilchen vorliegt.

5. Verfahren nach Anspruch 4, bei dem das aus dem ersten Reaktor austretende Medium vor dem Eintragen in den nachfolgenden Reaktor durch ein Filter geht.

6. Verfahren nach einem der Ansprüche 1 bis 5, bei dem man das in den nachfolgenden Reaktor eintretende Medium vor dem Eintragen in den nachfolgenden Reaktor zunächst einer Entspannung unterwirft.

7. Verfahren nach einem der Ansprüche 1 bis 6, bei dem man das in den nachfolgenden Reaktor eintretende Medium vor dem Eintragen in den nachfolgenden Reaktor zunächst einer Behandlung zur Abtrennung des gebildeten Oxirans unterwirft.

8. Verfahren nach Anspruch 7, bei dem es sich bei der Abtrennungsbehandlung um eine Destillation handelt.

9. Verfahren nach einem der Ansprüche 1 bis 8, bei dem es sich bei dem Oxiran um Epichlorhydrin, bei dem Olefin um Allylchlorid, bei der Peroxidverbindung um Wasserstoffperoxid und bei dem Lösungsmittel um Methanol handelt und der Katalysator TS-1 enthält.

10. Verfahren nach einem der Ansprüche 1 bis 8, bei dem es sich bei dem Oxiran um Propylenoxid, bei dem Olefin um Propylen, bei der Peroxidverbindung um Wasserstoffperoxid und bei dem Lösungsmittel um Methanol handelt und der Katalysator TS-1 enthält.

11. Verfahren nach einem der vorhergehenden Ansprüche, bei dem man zwischen dem ersten und dem zweiten Reaktor keine erhebliche Lösungsmittelmenge zusetzt.

12. Verfahren nach einem der vorhergehenden Ansprüche, bei dem man dem zweiten Reaktor keine erhebliche zusätzliche Lösungsmittelmenge zusetzt.

13. Verfahren nach einem der vorhergehenden Ansprüche, bei dem man drei hintereinandergeschaltete Reaktoren verwendet.
